Europäisches Patentamt

European Patent Office

Office européen des brevets

(11) Publication number: **0 535 841 A1**

# EUROPEAN PATENT APPLICATION

(21) Application number: **92308595.5**

(22) Date of filing: **22.09.92**

(51) Int. Cl.5: **A61K  31/485**

(30) Priority: **04.10.91 GB 9121204**

(43) Date of publication of application:
**07.04.93 Bulletin  93/14**

(84) Designated Contracting States:
**AT BE CH DE DK ES FR GB GR IE IT LI LU MC NL PT SE**

(71) Applicant: **Euroceltique SA**
**122 Boulevard de la Petrusse**
**Luxembourg(LU)**

(72) Inventor: **Leslie, Stewart Thomas**
**4 Babraham Road**
**Cambridge(GB)**
Inventor: **Malkowska, Sandra Therese Antoinette**
**21 Broadway**
**Wilburton, Ely, Cambridgeshire(GB)**
Inventor: **Miller, Allan John**
**Weston Green, Thames Ditton**
**Surrey(GB)**
Inventor: **Miller, Ronald Brown**
**Bruderholzallee 191**
**CH - Basel 4059(CH)**

(74) Representative: **Lamb, John Baxter**
**MARKS & CLERK 57-60 Lincoln's Inn Fields**
**London WC2A 3LS (GB)**

(54) **Use of a combination of iburofen and codeine for the treatment of pain.**

(57)  The use of a combination of ibuprofen and codeine in the ratio 15:1 to 25:1 ibuprofen to codeine in the treatment of pain associated with chronic medical conditions. The ratio of ibuprofen to codeine is preferably 20:1. A preferred unit dose comprises 300mg ibuprofen and 20mg codeine phosphate (equivalent to 14.8mg codeine).

The present invention relates to a combination of ibuprofen and codeine and its use in the treatment of pain of chronic medical conditions.

Ibuprofen is a well know peripherally acting non-steroidal anti-inflammatory drug which also shows analgesic properties.

Codeine is a centrally acting opioid agonist with analgesic properties.

The effectiveness of ibuprofen-codeine combinations in the acute pain models of episiotomy and dental extraction has been investigated. Whilst it has been shown, for example, that multiple doses of a combination of ibuprofen (400mg) and codeine phosphate (30mg or 60mg) in the treatment of pain following dental surgery offer greater analgesic effect than placebo or codeine alone the same study found no significant difference over ibuprofen (Giles A D, Pickvance N J, Clinical Trial, 1985, 22, 300-313). Other studies showing no significant benefit for ibuprofen-codeine combinations compared to ibuprofen alone have been reported (see for example, Cooper et al, Pharmacotherapy, 1982, 2, 162-167; Giles et al, J Oral Maxillofac. Surg., 1986, 15, 727-732; Norman et al, Clinical Therapeutics, 1985, 7, 549-554).

EP 0388125-A describes pharmaceutical compositions comprising from 100 to 600mg of ibuprofen and from 12 to 40mg of codeine of use in the treatment of acute pain. In this case the analgesic effect of a combination of ibuprofen (400mg) plus codeine (20mg) was reported to be greater than from a single dose of ibuprofen alone in the treatment of pain following dental surgery.

The single administration of two tablets containing ibuprofen (300mg) and codeine phosphate (20mg) however was reported to offer no advantages over the use of ibuprofen alone in the treatment of dental extraction pain (Walton GM, Rood JP, British Dental Journal, 1990, 169(8) 245-247).

EP-A-0068838 discloses the separate or simultaneous administration of a narcotic analgesic and ibuprofen or flurbiprofen in the management of severe to moderate pain.

The use of compositions comprising 30-50 parts by weight ibuprofen and 1.5-4 parts by weight codeine in the treatment of pain is disclosed in EP-A-0413171.

Pharmaceutical compositions comprising a narcotic analgesic with a non-steroidal anti-inflammatory carboxylic acid derivative for the relief of mild to severe pain and for the treatment of inflammation in musculo-skeletal disorders are described in EP-A-0220805. There is no suggestion of synergy.

We have now surprisingly found that the combination of ibuprofen and codeine is particularly advantageous in the treatment of the pain of chronic medical conditions.

The present invention therefore provides the use of ibuprofen or a pharmaceutically acceptable salt thereof and codeine or a pharmaceutically acceptable salt thereof, the ratio of ibuprofen to codeine being in the range 15:1 to 25:1 for the manufacture of a medicament for the treatment of pain of chronic medical conditions.

In a preferred embodiment the ratio of ibuprofen to codeine in medicaments according to the present invention is 20:1.

Preferably medicaments according to the invention contain ibuprofen and codeine or their pharmaceutically acceptable salts as the only active ingredients.

The medicament is conveniently administered to give a daily dosage comprising from 300 to 2400mg (expressed as the weight of the free acid) of ibuprofen or a pharmaceutically acceptable salt thereof and from 14.8 to 281.2mg (expressed as the weight of the anhydrous free base) of codeine or a pharmaceutically acceptable salt thereof. Preferably an effective amount of the medicament is administered as a unit dose.

In a preferred embodiment the medicament is administered in a dose comprising 600mg (expressed as the weight of the free acid) of ibuprofen or a pharmaceutically acceptable salt thereof and 29.6mg (expressed as the weight of the anhydrous free base) of codeine or a pharmaceutically acceptable salt thereof and conveniently twice a day.

A preferred unit dose formulation comprises 300mg ibuprofen (or an equivalent amount of a pharmaceutically acceptable salt thereof) and 14.8mg codeine (or an equivalent amount of a pharmaceutically acceptable salt thereof). Conveniently one to four unit doses are administered at 12-hourly intervals.

The medicament according to the present invention is useful in treating pain associated with chronic medical conditions such as osteoarthritis, rheumatoid arthritis, ankylosing spondylitis, sero-negative arthropathies, bursitis, capsulitis of the shoulder, tendinitis, tenosynovitis, cancer. Medicaments according to the invention are particularly effective in treating the pain of arthritis, especially osteoarthritis.

Suitable pharmaceutically acceptable salts are those conventional in the art. Suitable pharmaceutically acceptable salts of ibuprofen include the sodium salt. Suitable pharmaceutically acceptable salts of codeine include the hydrochloride, acetate, salicylate and sulphate with the phosphate being particularly preferred. The preferred combination of active ingredients for use according to the invention is ibuprofen and codeine phosphate. It will be appreciated that codeine phosphate is preferably in the form of the hemihydrate.

Medicaments according to the present invention may be prepared by combining the active ingredients using conventional pharmaceutical techniques and are conveniently adapted for administration by any convenient route conventional in the art. Medicaments according to the invention are preferably adapted for oral adminstration.

The ibuprofen and codeine can be administered in the same dosage unit. Alternatively they can be prepared in separate dosage units to be administered at the same time. Different forms of dosage units can be used for each component.

For oral administration medicaments according to the invention may suitably take the form of tablets, capsules, granules, spheroids, powders or liquid preparations.

Tablets and capsules for oral administration may be prepared by conventional techniques with pharmaceutically acceptably excipients such as binding agents (for example pre-gelatinised maize starch or hydroxypropylmethylcellulose); fillers (for example lactose, microcrystalline cellulose or calcium phosphate), lubricants (such as magnesium stearate, talc or silica), disintegrants, wetting agents, colourants and flavourings. The tablets may be coated according to well known methods in the art.

In a preferred embodiment the medicament according to the invention is formulated as a bilayer tablet, especially of the type described in EP-A-0220805.

The ibuprofen and codeine components in medicaments according to the invention may be present in controlled release or normal release form. Preferably the ibuprofen component is present in controlled release form. Suitable materials for inclusion in a controlled release matrix include, for example

(a) Hydrophilic or hydrophobic polymers, such as gums, cellulose esters, cellulose ethers, protein derived materials, nylon, acrylic resins, polylactic acid, polyvinylchloride, starches, polyvinylpyrrolidones, cellulose acetate phthalate. Of these polymers, cellulose ethers especially substituted cellulose ethers such as alkylcelluloses (such as ethylcellulose), $C_{1-6}$ hydroxyalkylcelluloses (such as hydroxypropylcellulose and especially hydroxyethyl cellulose) and acrylic resins (for example methacrylates such as methacrylic acid copolymers) are preferred. The controlled release matrix may conveniently contain between 1% and 80% (by weight) of the hydrophilic or hydrophobic polymer.

(b) Digestible, long chain ($C_8$-$C_{50}$, especially $C_8$-$C_{40}$), substituted or unsubstituted hydrocarbons, such as fatty acids, hydrogenated vegetable oils such as Cutina (Trade Mark), fatty alcohols (such as lauryl, myristyl, stearyl, cetyl or preferably cetostearyl alcohol), glyceryl esters of fatty acids for example glyceryl monostearate mineral oils and waxes (such as beeswax, glycowax, castor wax or carnauba wax). Hydrocarbons having a melting point of between 25°C and 90°C are preferred. Of these long chain hydrocarbon materials, fatty (aliphatic) alcohols are preferred. The matrix may contain up to 60% (by weight) of at least one digestible, long chain hydrocarbon.

(c) Polyalkylene glycols. The matrix may contain up to 60% (by weight) of at least one polyalkylene glycol.

A suitable matrix comprises one or more cellulose ethers or acrylic resins, one or more $C_{12}$-$C_{36}$, preferably $C_{14}$-$C_{22}$, aliphatic alcohols and/or one or more hydrogenated vegetable oils.

A particularly suitable matrix comprises one or more alkylcelluloses, one or more $C_{12-36}$, (preferably $C_{14}$-$C_{22}$) aliphatic alcohols and optionally one or more polyalkylene glycols.

Preferably the matrix contains between 0.5% and 60%, especially between 1% and 50% (by wt) of the cellulose ether.

The acrylic resin is preferably a methacylate such as methacrylic acid copolymer USNF Type A (Eudragit L, Trade Mark), Type B (Eudragit S, Trade Mark), Type C (Eudragit L 100-55, Trade Mark), Eudragit NE 30D, Eudragit E, Eudragit RL and Eudragit RS. Preferably the matrix contains between 0.5% and 60% by weight, particularly between 1% and 50% by weight of the acrylic resin.

In the absence of polyalkylene glycol, the matrix preferably contains between 1% and 40%, especially between 2% and 36% (by wt) of the aliphatic alcohol. When polyalkylene glycol is present in the oral dosage form, then the combined weight of the aliphatic alcohol and the polyalkylene glycol preferably constitutes between 2% and 40%, especially between 2% and 36% (by wt) of the matrix.

The polyalkylene glycol may be, for example, polypropylene glycol or, which is preferred, polyethylene glycol. The number average molecular weight of the at least one polyalkylene glycol is preferably between 200 and 15000 especially between 400 and 12000.

The ibuprofen containing controlled release matrix can readily be prepared by dispersing the active ingredient in the controlled release system using conventional pharmaceutical techniques such as wet granulation, dry blending, dry granulation or coprecipitation.

Liquid preparations for oral administration may be in the form of, for example, solutions, syrups or suspensions, or they may be presented as a dry product for constitution with water or some other suitable vehicle before use. Such liquid preparations may be prepared by methods conventional in the art and may

EP 0 535 841 A1

contain pharmaceutically acceptable additives such as suspending agents, emulsifying agents, flavouring or colouring agents.

In a further aspect of the present invention also provides a method of treatment of chronic pain comprising administration of a medicament comprising ibuprofen or a pharmaceutically acceptable salt thereof and codeine, the ratio of ibuprofen and codeine being in the range of 15:1 to 25:1 or a pharmaceutically acceptable salt thereof.

The invention further provides a unit dose formulation for use in the treatment of chronic pain comprising the equivalent of 300mg ibuprofen and 14.8mg codeine.

In order that the invention may be more fully understood the following example is given by way of illustration only.

Example 1

Bilayer tablets having the following formulation were prepared:

|  | mg/tablet |
|---|---|
| **Ibuprofen layer** | |
| Ibuprofen | 300.0 |
| Microcrystalline cellulose | 50.1 |
| Lactose (anhydrous) | 50.1 |
| Hydroxyethylcellulose | 5.0 |
| Hydroxypropylmethylcellulose (5 cps) | 9.5 |
| Ponceau 4R Al. lake (E 124) | 4.3 |
| Purified Water | N.D. |
| Cetostearyl alcohol | 10.0 |
| Purified talc | 4.0 |
| **Codeine phosphate layer** | |
| Codeine phosphate | 20.0 (equivalent to 14.8mg anhydrous base) |
| Microcrystalline cellulose | 300.0 |
| Pregelatinised maize starch | 30.0 |
| Povidone (K30) | 14.0 |
| Purified Water | N.D. |
| Total tablet weight (mg) | 797.0 |

The ibuprofen, microcrystalline cellulose, anhydrous lactose, hydroxyethylcellulose, hydroxypropyl methyl cellulose and Ponceau 4R Al. lake were dry blended in a high speed mixer. Purifed water was added. The whole was then granulated, dried in a fluid bed dryer and sieved through a 1.6mm (12 mesh) screen. After sieving the granules were again dried and sieved through a 1.25mm (16 mesh) screen.

4

Molten cetostearyl alcohol was added to the granules and granulated. The coated ibuprofen granules were cooled and blended with purified talc.

Codeine phosphate, microcrystalline cellulose (260mg) and pregelatinised maize starch were dry blended in a high speed mixer. Povidone was dissolved in water and the solution together with further water was added to the codeine mixture and the whole was granulated. The granules were dried in a fluid bed dryer, sieved through a 0.9mm (20 mesh) screen, dried again and then blended with microcrystalline cellulose (40mg).

The ibuprofen and codeine phosphate granules were compressed into bilayer tablets having a controlled release ibuprofen layer and a normal release codeine layer using a rotary tablet machine with a 18.6 X 7.5mm capsule shape punch.

Example 2

Ibuprofen layer

An ibuprofen layer having the following formulation was prepared by a wet granulation process.

|  | mg/tablet |
| --- | --- |
| Ibuprofen | 200.0 |
| Microcrystalline cellulose | 68.59 |
| Sodium Starch Glycollate | 45.00 |
| Hydroxypropylmethylcellulose (3 cps) | 8.16 |
| Erythrosine Al. lake, E127 | 3.25 |
| Purified Water | N.D. |

The ibuprofen, microcrystalline cellulose, sodium starch glycollate and hydroxypropylmethylcellulose were dry mixed and Erythrosine aluminium lake was added. Water was added and the mixture granulated.

Codeine Phosphate layer

|  | mg/tablet |
| --- | --- |
| Codeine Phosphate | 12.50 |
| Microcrystalline cellulose | 267.50 |
| Pregelatinised maize starch | 25.0 |
| Povidone (K30) | 10.0 |
| Purified Water | N.D. |

Codeine phosphate granules were prepared by a method analagous to that described in Example 1.

The codeine phosphate and ibuprofen granules were compressed to give a bilayer tablet having a normal release ibuprofen layer and a normal release codeine layer. The resulting tablet was coated using hydroxypropylmethyl cellulose as film former and propylene glycol as plasticiser.

Clinical Test Results

A double-blind placebo controlled four-part, crossover study was conducted in 69 patients with osteoarthritis. The patients were randomly allocated to receive a week's treatment in turn of each of controlled release ibuprofen 300mg/codeine phosphate 20mg tablets according to Example 1 (IC); controlled release ibuprofen 300mg tablets (I); codeine phosphate 20mg tablets (C); or placebo tablets (P). Patients took two tablets twelve hourly.

After a one week period on the first randomly allocated treatments, patients were crossed over at weekly intervals until all patients had received each of the four treatments. Forty nine patients completed all four treatment periods.

Patients recorded daily their pain on movement during the day (on a scale of 0 (no pain) to 3 (severe pain). The presence or absence of night time pain was also recorded.

Treatment comparisons were made using data only from those patients completing both treatments within each paired comparison. The paired treatments analysed were IC versus I, IC versus C, I versus P and C versus P.

Results

Day Pain

Results based on analysis of measurements on day 5 to day 7 of each leg of the study are presented below. The figures in the table represent the median score for each patient for the degree of pain over the three assessment days, for the four treatment regimens.

| Recorded day pain | Treatments | | | | Test Medication | |
|---|---|---|---|---|---|---|
| | IC | I | C | P | | |
| Severe | 3 | 5 | 10 | 10 | IC | Combination |
| Moderate | 21 | 27 | 21 | 23 | I | Ibuprofen |
| Mild | 21 | 13 | 14 | 17 | C | Codeine phosphate |
| None | 6 | 8 | 3 | 2 | P | Placebo |

The results from all three days treatments were ranked then added (None, mild, moderate and severe scored from their ranks based on frequency) to produce a summary statistic for each patient on each treatment regimen. With increasing efficacy against pain, this rank would decrease. The Wilcoxon signed rank test was applied between the treatment groups as a test for effect.

The following results were obtained:

Combination (IC) and Ibuprofen (I) were statistically significantly different at the 5% level. (Sample size $n = 48$). The combination of ibuprofen and codeine is established to be superior in controlling pain to ibuprofen alone.

Combination (IC) and Codeine phosphate (C) were highly statistically signficantly different at the 0.01% level. (Sample size $n = 43$). The combination of ibuprofen and codeine is established to be superior in controlling pain to codeine alone.

The Ibuprofen (I) treated group was not shown to be statistically significantly different from the Placebo treated group. (Tested at the 5% level with a sample size $n = 50$).

The Codeine phosphate treated group was not shown to be statistically significantly different from the Placebo treated group. (Tested at the 5% level with a sample size $n = 45$).

The above analyses establish that the combination of ibuprofen and codeine (IC) is superior at controlling pain to either of the constituent parts (I or C).

Night Pain

Results based on analysis of measurements on day 5 to day 7 of each leg of the study are presented below;

| FREQUENCY OF WAKING WITH PAIN | NUMBER OF PATIENTS BY TREATMENT | | | |
|---|---|---|---|---|
| | IC | I | C | P |
| None | 29 | 22 | 16 | 23 |
| One night | 9 | 9 | 11 | 3 |
| Two nights | 4 | 2 | 4 | 4 |
| Three nights | 11 | 20 | 20 | 22 |
| Data missing | 16 | 16 | 18 | 17 |

The results were summed for each patient and compared between treatments using the Wilcoxon signed rank test. The following results were obtained.

Combination (IC) and ibuprofen (I) were statistically significant different at the 1% level (Sample size $n = 50$). The combination of ibuprofen and codeine is established to be superior in controlling pain to ibuprofen alone.

Combination (IC) and codeine phosphate (C) were highly statistically significantly different at the 0.001% level (sample size n = 47). The combination of ibuprofen and codeine is established to be superior in controlling pain to codeine alone.

The ibuprofen (I) treated group was not shown to be statistically significantly different from the placebo treated group (tested at the 5% level with a sample size n = 50).

The codeine phosphate treated group was not shown to be statistically significantly different from the placebo treated group (tested at the 5% level with a sample size n = 47).

The above analyses establish that the combination (IC) is superior at controlling pain to either of the constituent parts (I and C).

From inspection of the data, it was apparent that the results for the combination were much better than expected from the other treatments.

A statistical method for testing if this observed result was significant was derived by assessing the magnitude of the benefit of the combination to the expected additive effect of the two constituent parts. By comparing this difference to zero a true assessment of the potentiation (synergy) can be made.

The magnitude of the effect was tested according to the method of Wilcoxon ("Non-parametrics, Statistical Methods based on Ranks" E.L.Lehmann, 1975, Holden-Day Inc.), and the magnitude of the potentiation over and above the additive effect was shown to be statistically signficantly superior at the 5% significance level.

| MEASURE | n | SYNERGY* | 95% CONFIDENCE INTERVAL | p VALUE |
|---|---|---|---|---|
| Day Pain | 41 | -0.32 | -0.60 to -0.04 | 0.02 |
| Night Pain | 45 | -0.67 | -1.09 to -0.25 | 0.0015 |

* IC + P-I-C

This surprising advantage over and above the simple additive effect of the drugs can be attributed to a synergistic effect of the combination of the two drugs.

## Claims

1. The use of ibuprofen or a pharmaceutically acceptable salt thereof and codeine or a pharmaceutically acceptable salt thereof for the manufacture of a medicament for the treatment of pain of chronic medical conditions, the ratio of ibuprofen to codeine being in the range 15:1 to 25:1.

2. The use according to claim 1 wherein the ratio of ibuprofen to codeine is 20:1.

3. The use according to claim 1 or 2 wherein the medicament is adapted for oral administration.

4. The use according to any one of claims 1 to 3 for the treatment of osteoarthritis.

5. The use according to any one of claims 1 to 4 wherein the medicament is administered in a dose comprising 600mg (expressed as the weight of the free acid) of ibuprofen or a pharmaceutically acceptable salt thereof and 29.6mg (expressed as the weight of the anhydrous free base) of codeine or a pharmaceutically acceptable salt thereof.

6. The use according to claim 5 wherein the medicament is administered twice daily.

7. The use according to any one of claims 1 to 5 wherein a unit dose of the medicament comprises 300mg (expressed as the weight of the free acid) of ibuprofen or a pharmaceutically acceptable salt thereof and 14.8mg (expressed as the weight of the anhydrous free base) of codeine or a pharmaceutically acceptable salt thereof.

8. The use according to any one of claims 3 to 7 wherein the medicament is in the form of a bilayer tablet.

9. The use according to any one of claims 1 to 8 wherein the ibuprofen containing component is present in controlled release form.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 220 805 (EUROCELTIQUE SA) <br> * Whole document, especially page 2, lines 11-13; page 5, lines 5-10; claims 3,5 * | 1-9 | A 61 K 31/485 |
| D,X | EP-A-0 413 171 (DOLORGIET GmbH & CO. KG) <br> * Abstract; page 4, lines 1-45; page 5, lines 1-25; page 6, line 47 - page 7, line 16 * | 1-4,6 | |
| D,X | EP-A-0 388 125 (BEECHAM GROUP PLC) <br> * Abstract; page 2, line 35 - page 3, line 32 * | 1-3,5,7 | |
| X | EP-A-0 274 845 (THE BOOTS CO. PLC) <br> * Page 2, lines 9-12; page 3, lines 18-56 * | 1-4,6 | |
| X | GB-A-2 203 338 (ALZA CORP.) <br> * Abstract; page 3, line 22 - page 5, line 1; page 17, line 5 - page 18, line 19 * | 1-9 | TECHNICAL FIELDS SEARCHED (Int. Cl.5) <br><br> A 61 K |

The present search report has been drawn up for all claims

| Place of search | Date of completion of the search | Examiner |
|---|---|---|
| THE HAGUE | 17-12-1992 | MAIR J. |